# EUROPEAN PATENT APPLICATION

(11) **EP 2 050 462 A1**
(43) Date of publication of application: **22.04.2009**
(21) Application number: 07020401.1
(22) Date of filing: 18.10.2007
(51) Int. Cl.: A61K 38/49, A61P 9/10

(54) **Improved treatment of stroke patients**

(71) Applicant: PAION Deutschland GmbH, 52062 Aachen (DE)
(72) Inventor: Al-Rawi, Yasir, Alaa, Shafeek, Sharjah (AE)
(74) Representative: Von Renesse, Dorothea

(57) **Abstract**

A method for treating a stroke patient with thrombolysis, whereas prior treatment the patient is diagnosed for exhibiting cerebral tissue at risk and a cerebral artery occlusion.

## Description

The invention relates to distinct groups of patients for thrombolysis in stroke treatment.

Stroke is the third leading cause of death, after cardiovascular disease and cancer. Each year, stroke is diagnosed in 750,000 patients and contributes to nearly 168,000 deaths in the United States only. Stroke has a high personal and social impact because of the severe disability that the disease causes.

The administration of thrombolytics - such as plasminogen activators - to the patient in acute ischemic stroke therapy aims to rescue the "tissue at risk" (which is sometimes in the scientific literature also referred to as "penumbra") and to reduce the final infarct size, thereby improving patient clinical outcome. Presently available and approved regimes for intravenous thrombolytic therapy are based on the time interval after the onset of the stroke symptoms. In other words, only patients with a stroke onset no later than 180 min (3 hours) before treatment are considered to be treatable with thrombolytics. Before the treatment, these patients undergo non-contrast cerebral computed tomography (CT) evaluations in order to confirm the absence of cerebral hemorrhage and the absence of a cerebral hypodensity encompassing more than one third of the middle cerebral artery (MCA) territory. They are also evaluated to exclude contra-indications for thrombolysis constituting risk factors for hemorrhage (e.g. a malignant tumors, recent trauma, recent surgery, a brain tumor, a vascular malformation or an aneurysm).

However, even with such restrictive criteria, thrombolysis is associated with a significant risk of intracranial hemorrhage (ICH), which occurs up to 15% of all treated patients. The clinical benefit of the currently available thrombolytic therapy does not compensate for this risk. Thus, selection of patients to be included in a thrombolysis protocol according to the current criteria is not sufficiently satisfactory.

Accordingly, it is the objective of the present invention to suggest novel patient subgroups which will benefit from stroke treatment and therewith exclude those patients who are not assumed to profit from thrombolysis. Therewith the overall efficacy and safety of thrombolytic stroke therapy should be increased.

It has now been found by the inventors that the selection of patients for thrombolytic therapy only on the basis of individual imaging of stroke patients and time lapse since stroke onset bears the risk of including patients with only minor or transient stroke events into the treatment, and therewith expose these patients to the risk of ICH without an underlying medical need. Furthermore it has been found, that the number of patients eligible for stroke treatment can reasonably be limited to those patients, who exhibit a stroke event, which cannot be overcome by self-healing capacities and thus need medical treatment. These stroke events can be categorized as being educible by certain properties as outlined below. Accordingly in one embodiment of the invention distinct subgroups of patients are selected for the treatment with thrombolytics.

According to the invention the selected patient subgroups will undergo a process of individual imaging before treatment for assessing the possible tissue at risk which indicates potentially salvageable brain tissue. According to further embodiments of the invention these patients can be treated with a bolus injection of a plasminogen activator. In case desmoteplase is applied, in one embodiment of the invention a bolus of either about 90 or about 125 microgram plasminogen activator per kg body weight (e.g. desmoteplase) can be administered. The treatment can be initiated later than three hours after stroke onset.

### Background of the invention

The target tissue of thrombolytic therapy is the so called tissue at risk. The pathopysiological rationale behind this is as follows:

Immediately after occlusion of an artery supplying the brain, the regional cerebral blood flow (rCBF) decreases. Brain tissue with critically low perfusion, referred to as "ischemic tissue", first loses function and finally integrity due to a lack of glucose and oxygen. The area where the integrity of brain tissue is largely lost is known as the "infarct core" and develops within the first minutes of vessel occlusion at the center of the ischemic area. This infarct core is characterized by irreversible neuronal cell damage and is surrounded by ischemic, but still salvageable tissue at risk of infarction. The tissue at risk is also referred to as the "penumbra".

Depending on the time lapse since stroke onset and the severity of cerebral ischemia, the quantity of collateral flow, and the metabolic status of the patient, the tissue at risk will eventually also lose its structural integrity and thus progress to infarction. In the case of persistent vessel occlusion, the core of infarction expands over time until nearly all of the tissue at risk has progressed to infarction. However, in the case of early recanalization of the feeding vessel, the ischemic changes within the tissue at risk are reversible, and it can potentially be salvaged. Therefore, the tissue at risk is the target of current thrombolytic therapy.

Although the above-mentioned factors for the development of infarction as such are known, there is a lack of understanding of possible interactions and consequences. Accordingly, a convincing concept for the selection of treatable patient groups acceptable for the clinical routine use is still missing. In order to minimize the risk of hemorrhagic transformation, the only thrombolytic treatment presently approved is strictly limited to a maximum of 3 hours time from the onset of stroke symptoms.

### Summary of the invention

In contrast to the present thrombolytic therapy, the invention is based on the one hand on the individual assessment (diagnosis) of the tissue at risk (penumbra) of the patient, regardless of the time lapse after stroke onset. On the other hand the invention is based on the selection (diagnosis) of stroke patients suffering from a stroke which is due to an occlusion within a cerebral blood vessel. In one embodiment the occlusion is detectable by means of an imaging tool.

As used for the purpose of the invention the term "occlusion" is defined as any stricture or narrowing of a blood vessel which results in a reduced blood flow of the tissue distal thereof compared to the healthy or normal blood vessel. The occlusion can either be partial or complete. Hence, the term occlusion encompasses also a stenosis, i.e. an abnormal narrowing of a blood vessel still allowing distal perfusion.

According to the invention any imaging tool can be applied which results in the visualization of the inner opening of structures filled with blood and therewith enables the identification of an arterial occlusion. Possible imaging modalities include MRI or CT and further developments or modifications thereof; however without being limited to it. The visualization of blood vessels can also be referred to as angiography. Various methods for the further evaluation of MRI or CT images are known to the person skilled in the art (e.g. MTT, TTP or Tmax as post processing maps).

In a further embodiment of the invention, the occlusion is localized in a cerebral artery, in particular the middle cerebral artery (MCA), the anterior cerebral artery (ACA) and/or the posterior cerebral artery (PCA) including all of their branches, in particular M1 and/or M2.

Yet another embodiment of the invention is directed to a patient subgroup selected fir thrombolysis with an occlusion at baseline which is describable by a TIMI grade of below 3. In further embodiments the TIMI grade of occlusion is 2 or 1 or less. The grade of occlusion can also be 0, meaning complete occlusion.

The TIMI scale (Thrombolysis in Myocardial Infarction scale) was originally developed for the assessment of arterial occlusions in myocardial infarction and encompasses 4 grades as follows:
- grade 3:: normal blood flow
- grade 2:: artery entirely perfused but blood flow delayed
- grade 1:: artery penetrated by contrast material but no distal perfusion
- grade 0:: complete occlusion of the vessel.

In a further embodiment of the invention the selected patient group suffers a stroke which can be described by an NIHSS score of at least 4. However, the invention can also pertain to stroke patients with a NIHSS score of at least 8.

According to one embodiment a group of patient is selected for stroke treatment, which is characterized by a TIMI grade of below 3 and an NIHSS score of at least 4 in a particular embodiment the TIMI grade is 2 and the NIHSS score is 8.

Hence, turning away from the fixed 3 hour time window of present therapies, according to the invention, patients *without* tissue at risk are not considered to be treatable, even if they present at the hospital within the 3 hour time-window currently approved for rt-PA; whereas patients *with* tissue at risk will be open to receive thrombolytic medication, even if they arrive at the hospital *later* than the 3 hours after stroke onset; assuming that they can further be characterized as having a cerebral artery occlusion.

According to the invention, imaging techniques can be used for the assessment of tissue at risk, since they make it possible to obtain deeper insights into pathophysiological parameters in ischemic stroke, Imaging techniques can identify patients with an ischemia exceeding the infarct core, and who thus constitute the target population of the thrombolysis therapy according to this embodiment of the invention. Thus, pathophysiologically-based imaging in order to assess the tissue at risk as well as the infarcted core is a way to determine whether a patient is susceptible to the treatment according to the invention. Any imaging technique capable of assessing the tissue at risk is suitable, such as e.g. MRI or CT; however without being limited to it.

Imaging can also be used to ensure patient safety by excluding individuals at high risk for post-treatment hemorrhage and those with low likelihood of benefit because there is no demonstrable tissue at risk. Risk factors, which on a regular basis may exclude the treatment according to the invention (though not always), are the evidence of intracranial hemorrhage (ICH), subarachnoid hemorrhage (SAH), arteriovenous malformation (AV), cerebral aneurysm or cerebral neoplasm. Furthermore, according to one embodiment of the invention, patients with an acute infarction involving more than approximately 1/3 of the territory of the middle cerebral artery (MCA) or substantially the entire territory of the anterior cerebral artery (ACA) and/or the posterior cerebral artery (PCA) can be excluded from treatment by the invention. In addition, patients with signs of Blood Brain Barrier (BBB) leakage represent a risk factor for thrombolytic therapy and - according to one embodiment of the invention - should be excluded.

Although the concept of individual imaging is not limited to a certain time window, it can be favorable to treat the patients within a time window of up to 9 hours from stroke onset; i.e. the treatment is possible even later than 3 hours after stroke onset.

As mentioned above the patient subgroups selected according to the invention suffer from a stroke which needs medical treatment. These subgroups are characterized by one or more clinical properties as outlined in detail below.

The efficacy of the stroke treatment according to the invention can be shown by assessing the difference of percentage change of the core lesion volume from pre-treatment imaging assessment to day 30 after treatment between the groups with active treatment (verum) and placebo or with the comparison of the clinical response rate at day 90.

In one embodiment of the invention the treatment of the selected stroke patients can comprise the administration of either about 90 or about 125 microgram of a plasminogen activator per kg bodyweight of the patient, in particular desmoteplase.

### Detailed description of the invention

As outlined above, in one aspect of the invention individual imaging is used to diagnose or identify (select) candidates for the thrombolytic (recanalization) therapy. Any suitable imaging tool can be used. For example, MRI is an imaging tool that can be applied, which can be performed with a diffusion-weighted sequence (DWI). Strong hyperintensity on DWI indicates a core lesion destined to infarction with or without therapeutic reperfusion. It is normally surrounded by a hypoperfused region measured with PWI (Perfusion Weight Imaging).

Some patients show a hyperintensity on DWI which covers nearly the entire volume of hypoperfused tissue identified by PWI. This "match" of lesion size on DWI and PWI indicates minimal tissue at risk. Patients selected according to one embodiment of the invention present a PWI lesion distinctively larger than the DWI lesion and thus present a "mismatch", that indicates a potentially salvageable region of tissue at risk (penumbra), The region of the tissue at rik favorably is by at least about 20% larger than the region of the core infarct. The tissue at risk can be located e.g. in the area of the middle cerebral artery (MCA), the area of anterior cerebral artery (ACA) or the area of posterior cerebral artery (PCA). In an embodiment of the invention, these groups of "mismatch" patients or patients with a penumbra, which is at least about 20% larger than the core infarct, are subject of the thrombolytic therapy according to the invention.

Furthermore, MRA (Magnetic Resonance Angiography) can be used in order to identify the site of vessel occlusion. In cases where vessel occlusion persists, the formerly salvageable tissue at risk will likely infarct. After early recanalization perfusion of the tissue at risk will be normalized leading to tissue salvage.

MR as an imaging tool is named as an example only. The penumbra (tissue at risk") identification is also possible e.g. with CT using the perfusion CT (PCT) method, or Positron Emission tomography (PET). Another example is ultrasound visualization.

The NIHSS is a systematic assessment tool that provides a quantitative measure of stroke-related neurologic deficit. The NIHSS was originally designed as a research tool to measure baseline data on patients in acute stroke clinical trials. Now, the scale is also widely used as a clinical assessment tool to evaluate acuity of stroke patients, determine appropriate treatment, and predict patient outcome, According to the NIHSS, parameters such as the level of consciousness, the eye movement, the facial palsy or the motor ability of arms or legs are assessed and subject to a pre-defined numerical scoring. On a regular basis a NIHSS score of 6 or below is considered as a rather light stroke, whereas a NIHSS score from 6 to approximately 15 is qualified as a stroke of medium severity. A score of 15 or more of the NIHSS scale indicates a rather severe stroke. Frequently a stroke of an NIHSS score of 20 or more is considered as being untreatable. However, notably the qualification of the severity of a stroke depends also on the individual assessment of the patient by the physician, which includes aspects of the overall clinical performance of the patient. According to the invention a baseline NHISS score of at least 8 is required.

As outlined above, imaging can be applied also the exclude certain patient groups from thrombolysis, namely in order to exclude certain risk factors. Accordingly in one embodiment of the invention the selected patient groups do not exhibit one or more of the following properties
o acute infarction involving more than around 1/3 of MCA or substantially the entire ACA and/or PCA territory
o evidence of ICH, SAH, AV malformation, cerebral aneurysm or cerebral neoplasm.

These patients are favorably treated within 3 to 9 hours after the onset of stroke symptoms.

The clinical outcome can e.g. be measured as a "clinical response rate" at day 90 after treatment, which e.g. is defined as having achieved one or more of the three parameters as follows:
i. at least 8 point NIHSS improvement or a final NIHSS score of 0-1 at day 90, e.g. an improvement from NIHSS score from 24 to 16 or to 0-1 (if the patient at baseline had a NIHSS score of 9 or below)
ii. a score in the modified Rankin Scale (mRS) of 0-2
iii. a Barthel Index between approximately 75 to approximately 100.

Furthermore, these groups of patients can show a reduction of the infarct core lesion volume at day 30 compared to the pre-treatment state (baseline).

The plasminogen activator used for the stroke treatment according to either one or both embodiments of the invention can be administered to the patient as a single bolus injection with a plasminogen activator concentration of either about 90 or about 125 micrograms per kg body weight of the patients. Hence the invention also pertains to the manufacture of a medicament (i.e. a certain dosage unit form) for treating selected stroke patients comprising a dosage, which allows the preparation of a ready-to-use formulation comprising either about 90 or about 125 micrograms/kg body weight. The dosage unit form can be, e.g. a solid such as a lyophilisate, or a liquid in a vial or ampul. In an embodiment the dosage unit form contains about 9.0 or 12.5 mg plasminogen activator such as e.g. Desmoteplase.

The patient selected according to the invention show a reduction of the infarct core lesion volume at day 30 compared to the pre-treatment state (baseline).

The thrombolytic treatment according to the invention can be performed with any plasminogen activator (PA). As used herein the term "plasminogen activator" refers to all substances - either naturally or synthetically provided, with human origin or non-human origin - which stimulate via the proteolytic activation of plasminogen to plasmin the clot lysis. Typical PAs known to the skilled person are, e.g., the tissue plasminogen activator (tPA), which is available in its recombinant form rtPA (alteplase), streptokinase or urokinase, and their respective derivates, fragments or mutants, which maintain the proteolytic activity (e.g. tenecteplase or reteplase for rtPA).

In an embodiment of the invention, a non-neurotoxic plasminogen activator is used, i.e., a plasminogen activator, which *per* se exhibits a substantially reduced potential to activate the NMDA type glutamate receptor. This plasminogen activator favorably is essentially non-activatable by beta-amyloid or prion protein and shows in the presence of fibrin an enhanced activity of more than about 550 fold, more than about 5500 fold, or more than about 10,000 fold, compared to the activity in the absence of fibrin. In yet an embodiment, the increase of activity of the PA in the presence of fibrin compared to its activity in the absence of fibrin is more than about 100,000. Since the increase in activity of rt-PA is about 550, in one embodiment of the invention a PA is used which has an approximately 180-200 fold higher fibrin specificity/selectivity compared to rt-PA.

The neurotoxicity can be assessed by methods known to the skilled person, e.g. with animal models in particular kainic acid models as described in detail in the international laid open WO03/037363. This model is further described in detail in Liberatore et al. (Liberatore, G.T.; Samson, A.; Bladin, C.; Schleuning, W.D., Medcalf, R.L. "Vampire Bat Salivary Plasminogen Activator (Desmoteplase)", Stroke, February 2003, 537-543) and Reddrop et al. (Reddrop, C.; Moldrich, R.X.; Beart, P.M.; Liberatore, G.T.; Howells, D.W.; Schleuning, W.D.; Medcalf, R.L., "NMDA-mediated neurotoxicity is potentiated by intravenous tissue-type-, but not vampire bat-plasminogen activator, and is enhanced by fibrin", Monash University Department of Medicine, Version November 20, 2003).

In yet another embodiment of the invention the PA has a plasma half-life of more than 2.5 min, more than 50 min, or more than 100 min.

In an embodiment of the invention, DSPA alpha 1 or PA with a biological activity and pharmacological properties essentially corresponding to DSPA alpha 1 are used. DSPA alpha 1 has a half-life of 138 min and a 105,000 fold increased activity in the presence of fibrin compared to its activity in the absence of fibrin.

DSPA alpha 1 is a plasminogen activator, which originally was isolated or derived from the saliva of *Desmodus rotundus* (*Desmodus* Salivary Plasminogen Activator). Within the saliva, four variants of DSPA had been isolated which, similarly to alteplase and urokinase, are composed of various conserved domains previously established in related families of proteins. The variants rDSPA alpha1 and rDSPA alpha2 exhibit the structural formula Finger (F), Epidermal Growth Factor (EGF) (sometimes also referred to as "(E)"), Kringle (K), Protease (P), whereas rDSPA beta and rDSPA gamma are characterized by the formulas EKP and KP, respectively. Subtle sequence differences and data from southern blot hybridisation analysis indicate that the four enzymes are coded by four different genes and are not generated by differential splicing of a single primary transcript.

The variant DSPA alpha 1 has an at least 70% structural homology to alteplase (rt-PA); the difference being that alteplase has two kringles (FEKKP), whereas DSPA alpha 1 has only one (FEKP). DSPA alpha 1 is a serine protease with 441 amino acids. Like other plasminogen activators (PA), DSPA alpha 1 activates plasminogen by catalysing the conversion of plasminogen into plasmin, which in turn breaks down the cross-linked fibrin abundant in blood clots.

DSPA alpha 1 has been found to have a high specificity for plasminogen-bound fibrin, high fibrin selectivity (defined by activation by fibrin relative to activation by fibrinogen), substantially no neurotoxicity, and negligible activation by beta-amyloid and human cellular prion protein, in addition to a long dominant half-life of more than 2 hours (see above).

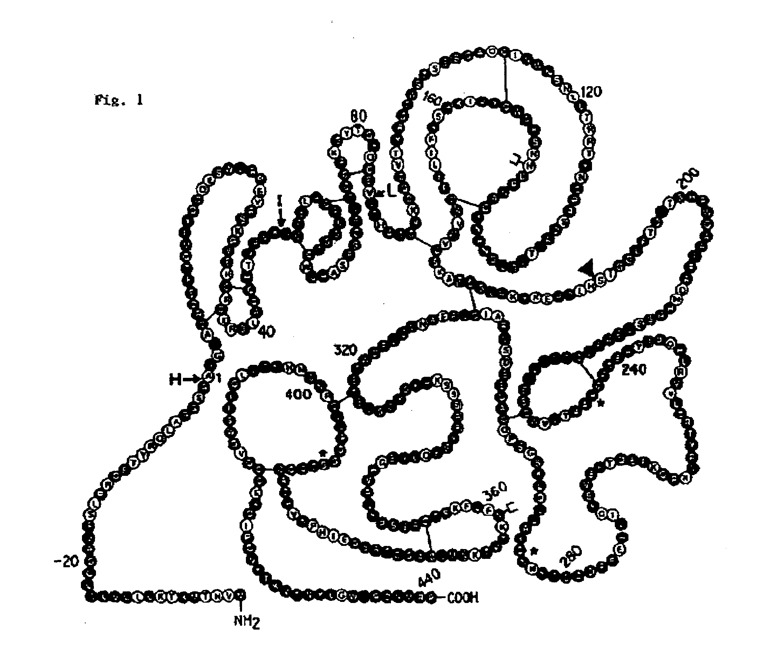

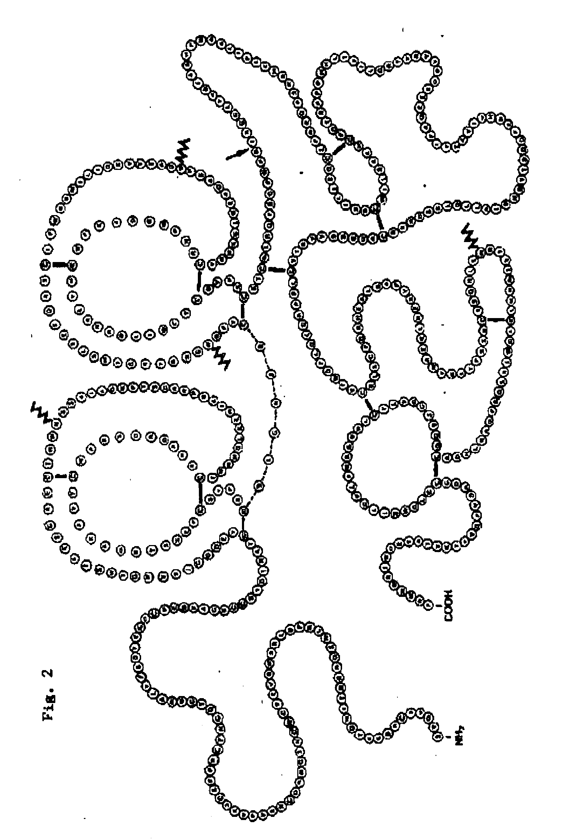

Recombinant DSPA alpha 1 can be obtained from Chinese hamster ovary cells containing a recombinant plasmid carrying the DSPA alpha 1 gene from *Desmodus rotundus*. **Fig.1** and **fig. 2** show the structures of DSPA alpha 1 and alteplase. The sequence of the mature DSPA alpha 1 is shown in **fig. 3****.**

The plasminogen activators from *Desmodus rotundus* and their recombinant forms were first disclosed in US patent Nos. 6,008,019 and 5,830,849. US 6,008,019 discloses the sequence data of DSPA alpha 1. Both patents are incorporated herein by reference in terms of the structure, properties and manufacture of plasminogen activators from *Desmodus rotundus*, in particular DSPA alpha 1. The recombinant manufacture and further processing is also subject of the EP 1 015 568 B1, which is also incorporated herein by reference for its disclosure of recombinant manufacture of DSPA alpha 1.

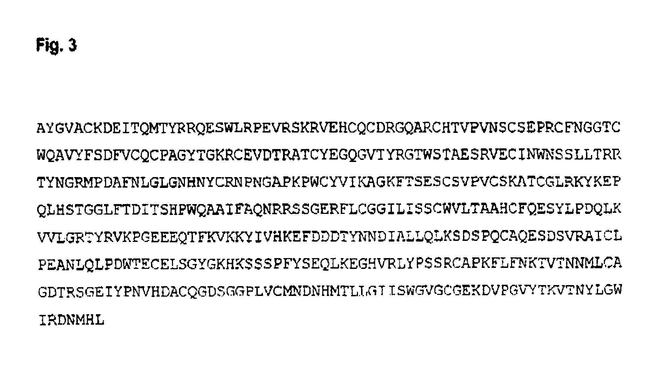

According to the present invention, the term "desmoteplase" is used for any plasminogen activator with identical or essentially the same biological activity of DSPA alpha 1 regarding the activation of plasminogen and its enhanced fibrin selectivity/specificity. In a further embodiment the fibrin selectivity is at least 180 fold compared to rt-PA. The PAs defined as desmoteplase according to the invention can be at least 80 or 90%, at least 95 %, or at least 98% identical to the amino acid sequence according to fig. 3 (DSPA alpha 1). The plasminogen activators can include microheterogeneities, e.g. in terms of glycosylation and/or N-terminal variations, which are merely due to production systems.

## Claims

1. Use of a plasminogen activator for the manufacture of a medicament for treating stroke in a patient, whereas said patient prior treatment is selected for exhibiting
a. cerebral tissue at risk and
b. a cerebral artery occlusion.

2. Use of a plasminogen activator according to claim 1, whereas the cerebral artery occlusion is localized in the MCA, ACA or PCA or branches thereof.

3. Use of a plasminogen activator according to claim 1 or 2, whereas the artery occlusion is in branch M1 or M2 of the MCA, ACA or PCA.

4. Use of a plasminogen activator according to one of the above claims, whereas the artery occlusion is of a TIMI grade of below 3, preferably 2 or less, most preferred 1 or less or 0.

5. Use of a plasminogen activator according to one of the above claims, whereas the tissue at risk is localised in the area of MCA, ACA or PCA.

6. Use of a plasminogen activator according to one of the above claims, whereas the patient exhibits a stroke of a NIHSS score of at least 4, preferably of at least 8.

7. Use of a plasminogen activator according to one of the above claims, whereas the artery occlusion and/or the tissue at risk is assessed prior treatment by individual imaging.

8. Use of a plasminogen activator according to one of the above claims, whereas the tissue at risk is at least about 20 % larger than the core infarct.

9. Use of plasminogen activator according to one or more of the above claims, whereas the patient is further **characterized by** one or more of the following properties at baseline:
a. the acute infarction does not involve more than about 1/3 of MCA or substantially the entire ACA or PCA territory and/or
b. the absence of ICH, SAH, AV malformation, cerebral aneurysm or cerebral neoplasm.

10. Use of a plasminogen activator according to one or more of the above claims, whereas the plasminogen activator is administered to the patient in a dosage of about 90 or about 125 microg/kg body weight.

11. Use of a plasminogen activator according to one or more of the above claims, whereas the plasminogen activator has an at least more than about 550 fold increased activity in the presence of fibrin compared to the activity without fibrin.

12. Use of a plasminogen activator according to one or more of the above claims, whereas the plasminogen activator
i. is essentially non-activatable by beta-amyloid and/or prion protein and/or
ii. is substantially non-neurotoxic and/or
iii. has a half-life of at least more than 2.5 min.

13. Use of a plasminogen activator according to one or more of the above claims, wherein the plasminogen activator has an at least more than about 5500 fold increased activity in the presence of fibrin compared to the activity without fibrin and has a half-life of at least more than about 50 min.

14. Use of a plasminogen activator according to one or more of the above claims, wherein the plasminogen activator is desmoteplase.

15. Use of a plasminogen activator according to one or more of the above claims, wherein the plasminogen activator
i. has an amino acid sequence according to fig. 3 or microheterogeneous forms thereof or
ii. is at least 80 %, more preferred 95 %, even more preferred 98 % identical to the amino acid sequence of fig. 3.

16. Use of a plasminogen activator according to one or more of the above claims, later than 3 hours after stroke onset.
